Europäisches Patentamt

European Patent Office

Office européen des brevets

(19)

(11) Numéro de publication: **0 248 694**
**B1**

(12) **FASCICULE DE BREVET EUROPÉEN**

(45) Date de publication du fascicule du brevet:
**27.12.89**

(51) Int. Cl.⁴: **G 01 N 33/28**

(21) Numéro de dépôt: **87401062.2**

(22) Date de dépôt: **11.05.87**

(54) Procédé de détermination de la composition d'un mélange d'hydrocarbures en fonction de la température d'ébullition de ses constituants.

(30) Priorité: **27.05.86 FR 8607691**

(43) Date de publication de la demande:
**09.12.87 Bulletin 87/50**

(45) Mention de la délivrance du brevet:
**27.12.89 Bulletin 89/52**

(84) Etats contractants désignés:
**BE DE ES GB IT NL**

(56) Documents cité:
**EP-A-0 026 012
US-A-3 379 202
US-A-4 012 291
US-A-4 568 426**

(73) Titulaire: **INSTITUT FRANCAIS DU PETROLE, 4,
Avenue de Bois- Préau, F-92502 Rueil- Malmaison
(FR)**

(72) Inventeur: **Favre, Anne, 30, Boulevard Robespierre,
F-78300 Poissy (FR)**
Inventeur: **Espitalie, Jean, 3, Allée des Acacias,
F-78110 Le Vésinet (FR)**
Inventeur: **Toulhoat, Hervé, 51, Avenue Charles
Tellier, F-78800 Houilles (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

EP 0 248 694 B1

**Description**

L'invention concerne un procédé de détermination de la composition d'un mélange d'hydrocarbures en fonction de la température d'ébullition de ses constituants. Plus particulièrement ce procédé permet d'effectuer rapidement une distillation analytique sensiblement complète des composés hydrocarbonés en présence et d'effectuer une mesure pondérale simultanée des produits pyrolysés et du carbone ou coke résiduel.

L'invention s'applique notamment au domaine du raffinage des produits lourds du pétrole (en particulier hydrotraitement et craquage catalytique), par exemple au contrôle des charges et des effluents en raffinerie.

Ces charges (mélanges d'hydrocarbures) peuvent être par exemple aussi bien des huiles lourdes brutes que des résidus issus de différents procédés de raffinage. On entend ici par huiles lourdes ou résidus des coupes de point d'ébullition initial à pression atmosphérique normale supérieur ou égal à 200°C.

Jusqu'à présent plusieurs méthodes étaient utilisées pour effectuer la distillation en laboratoire de ces produits lourds: les distillations vraies, analytique et préparative, et la distillation simulée par chromatographie en phase gazeuse. Des exemples de distillation analytique vraie sont décrits dans les normes ASTM D86 et ASTM D1160, par exemple. La distillation simulée consiste à séparer sur colonne chromatographique le produit lourd et à effectuer une intégration par tranche du chromatogramme en résultant. L'axe des temps étant étalonné par des n-alcanes de point d'ébullition connu, il est possible de transformer la courbe % quantité éluée, fonction du temps en % quantité vaporisée, fonction de la température, ce qui revient à une courbe de distillation (J.P. DURAND, N. PETROFF: Caractérisation des huiles lourdes et des résidus pétroliers, Symposium International LYON 25 - 27 juin 1984 (Actes) pages 200 - 205 Edition TECHNIP).

La principale limitation de ces deux types de distillation classique et simulée, est le point final de la distillation. La distillation classique est en effet limitée à un point final de 350°C à pression atmosphérique. Au dessus de cette température le produit est dégradé par craquage thermique.

La distillation simulée est limitée à une température de 550°C par la stabilité de la colonne. De plus, elle ne permet pas une quantification directe du résidu si tout l'échantillon ne s'est pas vaporisé, ni une quantification de chaque fraction éluée. C'est pourquoi il est nécessaire d'introduire plusieurs étalons internes (par exemple des n-alcanes) pour convertir le signal du détecteur en quantité d'étalons vaporisés et donc en quantité d'hydrocarbures évaporés.

Enfin, le temps d'analyse étalonnage compris, est long (environ 7 heures par échantillon), d'autant plus qu'il est nécessaire d'effectuer des blancs entre chaque nouvelle analyse afin de s'affranchir des effets de mémoire de la colonne chromatographique qui a tendance à retenir les produits les plus lourds et les plus polaires. Les risques d'encrassage et de bouchage de la colonne s'accentuent lorsque l'on veut traiter des produits lourds.

On peut certes éventuellement avoir recours à une distillation dite moléculaire, sous vide mais cette technique est très longue et difficile de mise en oeuvre et on ne peut obtenir qu'une quantité de distillat dans un intervalle de température donné et non pas une répartition en fonction des températures d'ébullition des produits distillés. C'est donc une méthode préparative et non analytique.

Par ailleurs, il existe des techniques de détermination du potentiel de cokéfaction, notamment par la mesure du carbone Conradson (norme ASTM D189). Le brevet US-4 568 426 décrit par exemple un appareil sophistiqué susceptible de reproduire avec précision et simplicité la mesure du carbone Conradson où l'échantillon est chauffé en atmosphère inerte, les produits gazeux vaporisés sont purgés en atmosphère inerte et le résidu pesé.

L'art antérieur est également décrit dans le brevet EP-A-0 026 012 (SHELL) où il est enseigné une méthode de détermination de la densité API d'une huile par vaporisation des composés volatils et pyrolysables et mesure de la quantité d'hydrocarbures dans une gamme de température, par exemple de 350°C à 750°C, par rapport à la quantité totale vaporisée.

Le brevet US-A-4 012 291 décrit aussi une méthode de mesure de la quantité d'oléfines présentes dans des hydrocarbures selon laquelle on ajoute une quantité mesurée d'hydrocarbures dans un four adapté à la vaporisation et on utilise un gaz vecteur pour le transport des hydrocarbures vaporisés dans une cellule de mesure à base de sels de mercure. On mesure ensuite la quantité totale d'oléfines présentes dans l'échantillon par intégration du signal.

Par ailleurs, l'art antérieur est notamment illustré par le brevet US-3 379 202.

Un des objets de l'invention est de remédier à ces inconvénients et notamment d'obtenir la courbe de distillation de l'échantillon (% distillé = f(T°C)), quel que soit le point d'ébullition final de la charge et en s'affranchissant de l'étalonnage interne.

Un second objet de l'invention est d'obtenir simultanément une distillation simulée de l'échantillon et un équivalent du carbone Conradson traduisant son potentiel de cokéfaction.

Enfin, un autre objet de l'invention est de déterminer un indice de pyrolyse qui est un paramètre dépendant du degré d'évolution de l'échantillon à travers soit son histoire géologique, soit une opération de raffinage.

Ces objectifs sont atteints par la mise en oeuvre d'un procédé de distillation simulée d'un échantillon d'hydrocarbures couplé notamment à une détermination d'un potentiel de cokéfaction, procédé que l'on caractérise par les étapes successives suivantes:

a) on imprègne une matrice poreuse stable et inerte par lesdits hydrocarbures dans un rapport pondéral de 0,01 à 1 mg d'hydrocarbures par milligramme de matrice,

b) on chauffe à température progressivement croissante et sous flux d'atmosphère inerte la matrice ainsi imprégnée jusqu'à une température $\theta_3$ comprise entre 500 et 600°C de façon à réaliser durant le temps de montée à ladite température une étape d'évaporation et une étape de pyrolyse sur au moins une partie desdits hydrocarbures et l'on produit un effluent d'évaporation, un effluent de pyrolyse et un résidu,

c) on mesure sur lesdits effluents au cours de ce chauffage et au moyen d'un détecteur d'hydrocarbures, au moins deux signaux $Q_1....Q_n$ et avantageusement une série de signaux successifs $Q_i$ représentatifs d'une quantité d'hydrocarbures, lesdits signaux $Q_1....Q_n$ correspondant à au moins deux intervalles de temps définis successifs de ce chauffage,

d) on réalise ensuite sur ledit résidu une combustion à une température $\theta_4$ supérieure à la température $\theta_3$, par exemple au plus égale à 1 200°C sous courant de gaz oxydant, ladite combustion produisant un effluent gazeux de combustion,

e) on mesure sur ledit effluent gazeux de combustion de l'étape d) au moyen d'au moins un détecteur approprié, un signal R représentatif de la quantité dudit résidu, et

f) on déduit desdites mesures des signaux $Q_1....Q_n$ et R la composition du mélange d'hydrocarbures en fonction de la température d'ébullition de ses constituants.

Selon une caractéristique de l'invention, dans l'étape b/ on peut chauffer ladite matrice imprégnée jusqu'à une température finale $\theta_2$ correspondant à ladite étape d'évaporation, comprise entre 330 et 370°C puis jusqu'à une température finale $\theta_3$ correspondant à ladite étape de pyrolyse, comprise entre 500 et 600°C et, dans l'étape c) on mesure au moins un signal $Q_1$ représentatif d'une quantité d'hydrocarbures correspondant à au moins un intervalle de temps défini de ladite étape d'évaporation et au moins un signal $Q_n$ représentatif d'une quantité d'hydrocarbures correspondant à au moins un intervalle de temps défini successif de ladite étape de pyrolyse.

Selon un autre mode de mise en oeuvre du procédé, on peut, par exemple, au moyen d'un détecteur spécifique du dioxyde de carbone, mesurer un signal représentatif d'une quantité de dioxyde de carbone contenu dans l'effluent gazeux de combustion et par conséquent, de la quantité de résidu. Mais, s'il est utile de tenir compte après l'étape de combustion de la présence d'oxydes de soufre ou d'azote dans une charge très soufrée et/ou très azotée, on peut combiner en série plusieurs détecteurs appropriés à ces oxydes ou utiliser un seul détecteur, multicanal à infrarouge par exemple, pour obtenir un signal R représentatif de la quantité de dioxyde de carbone, d'oxydes de soufre et d'azote et donc de la quantité dudit résidu.

Lorsque l'échantillon comprend des hydrocarbures de point d'ébullition final peu élevé ou de teneur en asphaltènes basse, on peut avantageusement ne pas réaliser l'étape de combustion et de mesure du signal R. Il est cependant préférable même dans ce cas particuliers de mettre en oeuvre l'ensemble du procédé, ne serait-ce que pour vérifier qu'il n'y a pas de résidu.

L'échantillon est imprégné sur une matrice. Celle-ci est sensiblement inerte vis-à-vis des réactions de déshydrogénation et de déhydrocyclisation des hydrocarbures; avantageusement, elle ne provoque pas plus de 30 % de conversion du n-heptane sous une pression de 10 bars, une température de 470°C, et une VVH égale à 1,5 $cm^3/cm^3/h$. La matrice est également stable: elle résiste à la température en atmosphère inerte et en atmosphère oxydante, de plus elle ne subit aucune transformation chimique susceptible d'augmenter l'intensité du signal de mesure.

La matrice est en général un oxyde non réductible (c'est-à-dire réfractaire à toute réduction sous hydrogène à 1 000°C), par exemple une alumine, une silice, une silice-alumine dont la teneur totale en métaux de transition sera inférieure à 1 000 ppm (parties par million). On peut prendre par exemple du sable de Fontainebleau. La porosité de la matrice est comprise entre 0,1 et 0,6 $cm^3/cm^3$ et de préférence entre 0,15 et 0,3 $cm^3/cm^3$. Sa surface spécifique est de 0,1 250 $m^2/g$ et de préférence de 0,2 $m^2/g$ à 100 $m^2/g$.

La présence de la matrice poreuse permet tout au long des différentes étapes de ménager une aire de contact élevée entre le matériau inerte et le produit lourd hydrocarboné. Grâce au flux de gaz inerte à travers cette matrice il est possible d'évaporer, sans dégrader par craquage, des produits de points d'ébullition égaux environ à 700°C.

La quantité d'hydrocarbures imprégnés sur la matrice est défini par un rapport pondéral compris entre 0,01 et 1 mg d'hydrocarbures par milligramme de matrice, ce qui permet une précision de mesure satisfaisante d'une part et ce qui évite la saturation du détecteur d'autre part. Avantageusement le rapport peut être compris entre 0,04 et 0,8 mg/mg, ce qui favorise la répétabilité de la mesure et d'excellents résultats ont été obtenus lorsque le rapport était compris entre 0,1 et 0,3 mg/mg; cette quantité d'hydrocarbures peut avoisiner par exemple 2 à 8 mg pour 10 à 50 mg de matrice, et de préférence 3 à 6 mg d'hydrocarbures pour 20 à 30 mg de matrice.

L'invention sera mieux comprise au vu des figures et exemples illustrant la mise en oeuvre du procédé parmi lesquels:

- la figure 1 illustre schématiquement les moyens de mise en oeuvre du procédé,
- les figures 2A et 2B représentent le diagramme d'évaporation, de pyrolyse et de combustion d'un échantillon de résidu non traité et d'un échantillon du même résidu hydrotraité, et
- la figure 3 montre les courbes de distillation simulée obtenues sur trois échantillons ainsi que les courbes obtenues selon l'art antérieur.

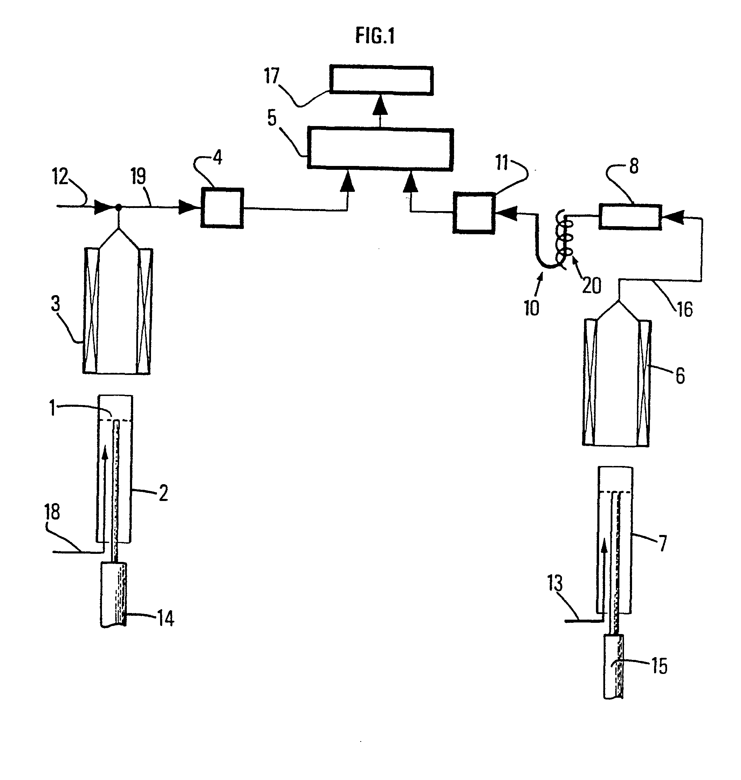

Selon la figure 1, le procédé se déroule de la façon suivante:

1°) Une fois imprégnée par l'échantillon, la matrice est mise dans une nacelle 1 prenant place au sommet d'un piston 2 mis en mouvement par un jeu de vérins 14. La nacelle 1 est placée dans un premier four 3

# EP 0 248 694 B1

dans lequel on effectue grâce à une alimentation 18 en gaz inerte, une évaporation sous flux de gaz inerte, par exemple azote, argon, hélium et de préférence de l'hélium pour avoir une meilleure réponse du détecteur 4. Le débit de gaz est de 20 à 100 ml/mn, de préférence 40 à 70 ml/mn. Le débit spécifique de gaz inerte rapporté à la quantité de matrice poreuse imprégnée est de 1 à 50 millilitres par milligramme et par minute et de préférence de 2 à 10 ml/mg/min.

Une programmation de température est réalisée:

a) un isotherme de $t_1$ minutes à la température initiale $\theta_1$, par exemple de 0,5 à 20 min, de préférence de 1 à 5 min est éventuellement appliqué. $\theta_1$ sera compris entre 50 et 200°C, de préférence de 80 à 120°C,

b) une montée en température à la vitesse v (v sera comprise entre 5 et 50°C/min, de préférence 8 à 15°C/min), jusqu'à la température de fin d'évaporation $\theta_2$ comprise entre 330°C et 370°C. Un nouvel isotherme à cette température $\theta_2$ de 0,5 à 20 min est appliqué. Les effluents de l'évaporation sont véhiculés par une ligne 19 alimentée en gaz inerte 12 et détectés au cours du temps par un détecteur 4 à ionisation de flamme préalablement étalonné avec un échantillon de référence. Les résultats sont intégrés par tranche (par exemple une intégration toutes les 1 à 120 s, de préférence toutes les 60 s) et conservés en mémoire par le microprocesseur 5. On obtient ainsi au moins un signal $Q_1$ en fonction d'au moins un intervalle de temps $\Delta t_1$ lors de l'étape d'évaporation.

2°) La même programmation de température est éventuellement appliquée jusqu'à la température $\theta_3$ comprise entre 500 - 600°C, de préférence 550°C. Un palier isotherme durant un temps $t_2$ de 0,5 à 20 min et avantageusement de 1 à 10 min est éventuellement appliqué. Cette montée en température s'effectue toujours sous gaz inerte afin de réaliser une pyrolyse.

Les effluents de la pyrolyse sont détectés au cours du temps par le détecteur d'hydrocarbures, par exemple un détecteur à ionisation de flamme 4, et les résultats sont intégrés comme précédemment et conservés en mémoire par le microprocesseur 5. On obtient alors au moins un signal $Q_n$ en fonction d'au moins un intervalle de temps $\Delta t_n$ ($\Delta t_n$ différent ou égal à $\Delta t_1$).

3°) La nacelle 1 contenant le résidu de pyrolyse est ensuite transférée par un autre piston 7 et un autre vérin 15 dans un deuxième four 6, avantageusement distinct du premier. On effectue une combustion en atmosphère oxydante grâce à une ligne 13 (air, $O_2$, mélange air/$O_2$ où le pourcentage d'oxygène représente plus de 20 % de la quantité totale) à une température $\theta_4$ supérieure à $\theta_3$ et comprise entre 550°C et 1 200°C, de préférence entre 600 et 800°C. Le temps de résidence à $\theta_4$ est compris entre 2 et 20 min, de préférence entre 5 et 10 min.

Le carbone organique présent dans le résidu se transforme en gaz carbonique et en monoxyde de carbone. Ce mélange passe par une ligne 16 dans un four à catalyse 8 chauffé à environ 400°C où se trouve un catalyseur oxydant (CuO par exemple) afin de transformer le monoxyde de carbone en gaz carbonique. Ce gaz carbonique est retenu sur un piège à azote liquide ou à adsorbant solide 10, puis détecté par un détecteur spécifique 11, par exemple un catharomètre, qui délivre un signal R. Cette mesure est conservée en mémoire dans le microprocesseur 5.

A partir du poids de gaz carbonique, on calcule le poids de carbone organique et on déduit le poids de résidu $m_r$ en prenant une valeur moyenne de la teneur en carbone (par exemple 0,83) de ce résidu.

L'intégration par tranche de la courbe d'évaporation ($Q_1$) et de la courbe de pyrolyse ($Q_n$) permet d'accéder au poids global évaporé $m_e$ et pyrolysé $m_p$ (grâce à un étalonnage préalable du détecteur transformant le signal en quantité d'hydrocarbures) donc au poids total de l'échantillon $m_t = m_e + m_p + m_r$.

Le rapport $m_r/m_t$ est directement corrélé au taux de résidu du carbone Conradson de l'échantillon.

Par ailleurs, grâce à un étalonnage par des n-alcanes il est possible de convertir l'échelle des temps (c'est-à-dire la température du four) en température d'ébullition.

Il est alors possible de reconstituer la courbe de distillation.

Cette méthode permet de réaliser trois mesures en une seule expérience:

— une évaporation qui donne la courbe de distillation simulée de l'échantillon jusqu'à une température sensiblement égale à 650°C, c'est-à-dire avec un point d'ébullition final supérieur à celui de la distillation analytique (T.B.P.) norme ASTM D2892 - 78 par exemple, ou norme ASTM D1 160, ou de la distillation simulée par chromatographie en phase gazeuse,

— une courbe de pyrolyse qui renseigne sur le potentiel de conversion,

— une caractérisation du résidu par la mesure d'un équivalent d'un carbone Conradson, qui évalue le potentiel de cokéfaction de l'échantillon.

Le temps d'analyse est court (environ 1 heure par échantillon), comparé aux autres méthodes (environ 7 heures pour une distillation simulée) et la mise en oeuvre en est simple; on s'affranchit de l'étalonnage interne indispensable en chromatographie d'où un gain de temps appréciable et indiscutable.

Les exemples qui suivent illustrent le procédé selon l'invention.

4

**Exemple 1**

Un résidu atmosphérique Safaniya (Arabie Saoudite) préalablement étuvé ou chauffé vers environ 80°C de façon à réduire sa viscosité dynamique à moins de 100 mm$^2$/s est homogénéisé par un moyen d'agitation et prélevé au compte-goutte par exemple. Une goutte calibrée d'environ 5 microlitres est déposée au contact de la matrice inerte qu'elle imprègne par capillarité. Cette matrice est constituée par environ 25 mg de sable de Fontainebleau (silice pure), de surface spécifique égale à 0,2 m$^2$/g et de porosité égale à 0,17 cm$^3$/cm$^3$. L'échantillon est introduit dans le four 3 sous balayage d'hélium.

Un isotherme de 1 minute à une température $\theta_1$ = 100°C est appliqué avant d'effectuer la montée en température du four de $\theta_1$ = 100°C à $\theta_2$ = 350°C à une vitesse de 10°C/min afin de réaliser l'évaporation. Les effluents de la vaporisation sont détectés en sortie du four sous courant d'hydrogène fourni par la conduite 12, par le détecteur à ionisation de flamme 4. Le signal est intégré toutes les 120 secondes et les résultats sont conservés en mémoire du microprocesseur 5. Un étalonnage permet d'obtenir ensuite la quantité d'hydrocarbures en fonction du signal obtenu.

La programmation de température est poursuivie sans interruption de $\theta_2$ = 350°C à $\theta_3$ = 550°C et se termine par un palier isotherme d'environ 1 minute à 550°C. Les effluents de pyrolyse sont détectés par le détecteur à ionisation de flamme 4. Le signal est intégré par tranche de 120 secondes et les résultats sont conservés en mémoire du microprocesseur 5.

L'échantillon 1 est ensuite introduit dans le second four 6 pour la phase d'oxydation, par exemple, par un passeur d'échantillons automatique non représenté sur la figure.

Au cours de celle-ci, la nacelle est poussée contre la partie supérieure du four 6, grâce au piston 7 afin que l'oxygène traverse l'échantillon. La température du four 6 est montée de la température ambiante à la température $\theta_4$ égale à environ 700°C le plus rapidement possible (1 à 2 min) puis un palier de 7 min à 700°C est appliqué. Le gaz carbonique et le monoxyde de carbone dégagés lors de la combustion passent dans un four à catalyse 8 rempli d'oxyde de cuivre CuO et chauffé à 400°C puis le gaz carbonique total est ensuite piégé sur un tamis 10 moléculaire 5 Å (5 x 10$^{-10}$ m).

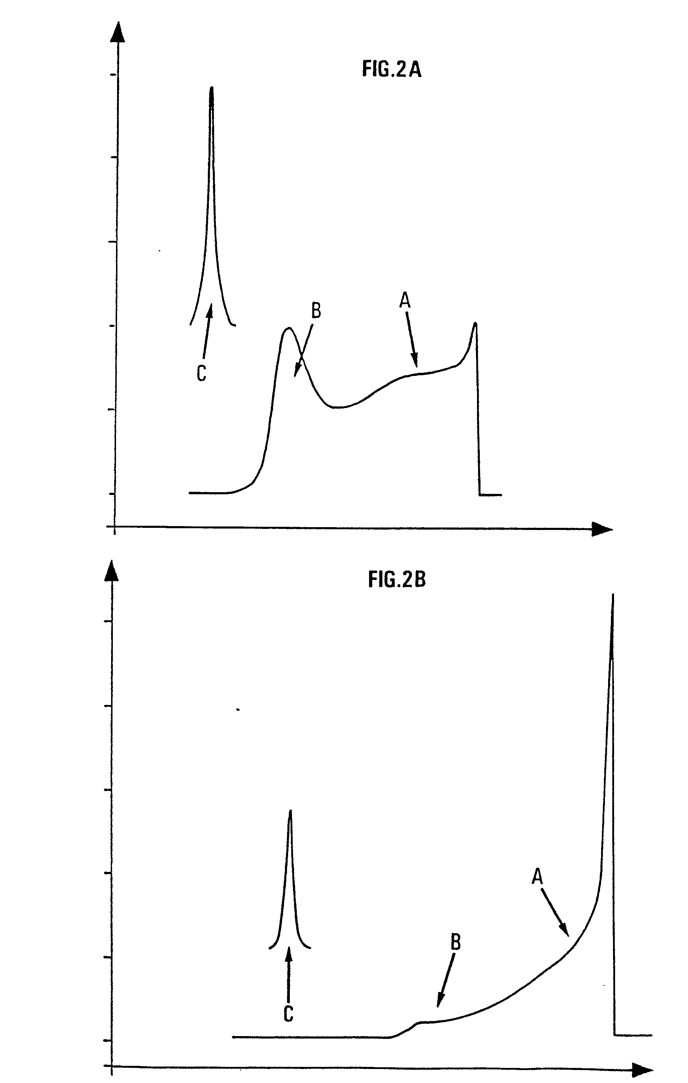

A la fin de la combustion, le piège 10 est purgé par des moyens de chauffage 20 et le gaz carbonique est détecté par le catharomètre 11. Les résultats sont conservés en mémoire du microprocesseur 5, puis l'ensemble des courbes d'évaporation A, de pyrolyse B et de combustion C, est reconstitué sur la figure 2A ci-dessous délivrée par l'imprimante 17.

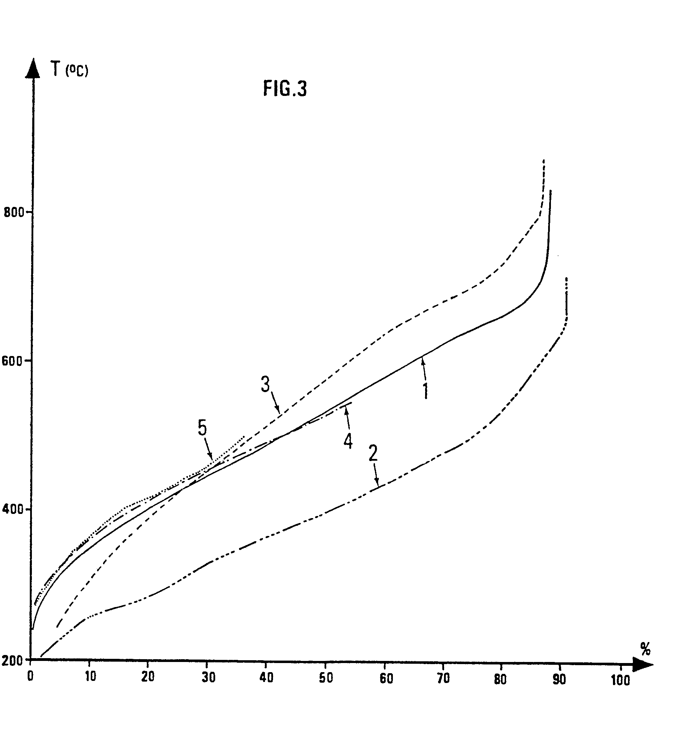

D'après le poids de gaz carbonique récupéré, on calcule le poids de carbone résiduel et en prenant comme valeur moyenne statistique de la teneur en carbone le facteur 0,83, on calcule le poids de résidu. Grâce à ce poids et à l'intégration par tranche de 120 s réalisé sur la courbe 1 de la figure 3 (en abscisse le pourcent poids cumulé, en ordonnée la température d'ébullition normale à pression atmosphérique), on reconstitue la courbe de distillation présentée dans le tableau I ci-dessous.

**Exemples 2 à 3**

L'échantillon de Safaniya a été hydrotraité puis analysé suivant le procédé de l'invention décrit selon l'exemple 1 (figures 2B et 3, courbe 2).

De la même façon, un échantillon d'une huile désasphaltée de Boscan (VENEZUELA) est analysé comme dans l'exemple 1 (courbe 3, figure 3). Les résultats sont présentés dans le tableau I.

**Tableau I**

| % poids cumulés T°C Ebullition | Résidu Safaniya | Résidu Safaniya hydrotraité | Boscan désasphalté |
|---|---|---|---|
| 238 | 0,5 | 5 | - |
| 260 | 1 | 10 | 6 |
| 280 | 2 | 17 | 7 |
| 300 | 4 | 24 | 9 |
| 320 | 6 | 28 | 11,5 |
| 340 | 8,5 | 32,5 | 14 |
| 360 | 12 | 39 | 16,5 |
| 380 | 15 | 45 | 18,5 |
| 400 | 20 | 50 | 22 |
| 420 | 23,5 | 56 | 25 |
| 440 | 28 | 61 | 28 |
| 460 | 33 | 65 | 31 |
| 480 | 38,5 | 70 | 35 |
| 500 | 42,5 | 75 | 38 |

**Tableau I** (cont.)

| % poids cumulés T°C Ebullition | Résidu Safaniya | Résidu Safaniya hydrotraité | Boscan désasphalté |
|---|---|---|---|
| 520 | 47,5 | 77,5 | 41 |
| 540 | 51 | 80 | 44 |
| 560 | 55 | 82,5 | 47 |
| 580 | 59 | 85 | 50 |
| 600 | 63 | 86,5 | 54 |
| 620 | 67,5 | 87,5 | 57 |
| 640 | 72,5 | 89,5 | 60 |
| Pyrolyse | 88 | 92,5 | 86,8 |
| Résidu | 100,0 | 100,0 | 100,0 |

**Exemples 4 et 5 selon l'art antérieur**

Sur la figure 3, ont été portées la courbe 4 représentant la distillation simulée du résidu Safaniya par chromatographie en phase gazeuse réalisée selon la référence à la publication J.P. DURAND 1984, citée ci-dessus, et la courbe 5 relative à la distillation TBP selon la norme ASTM D-2892-78.

La comparaison des courbes 1, 4 et 5 montre que les résultats sont sensiblement identiques jusqu'à une température de 450°C, que la distillation simulée par chromatographie est limitée à 530°C alors que le procédé selon l'invention permet de poursuivre la courbe de distillation jusqu'à 640°C. Au-delà de cette température, les phénomènes de craquage par pyrolyse interviennent.

**Exemples 6, 7, 8**

Un résidu atmosphérique Koweit a été analysé selon l'exemple 1 en présence d'une matrice de silice (sable de Fontainebleau). Ce même résidu a été analysé sur différents supports (alumine inerte ultrapure obtenue par calcination d'un gel d'alumine SB3 de la société CONDEA-CHEMIE et alumine contenant 3 000 ppm d'oxyde de fer). Les résultats m et la répétabilité (mesure de l'écart-type $\sigma$ à partir de 10 analyses) sont donnés dans le tableau II ci-dessous.

**Tableau II**

| | 600 % | Pyrolysable % | Résidu % |
|---|---|---|---|
| Silice m (sable de $\sigma$ Fontainebleau) | 43 2,5 | 47 2,5 | 10 2,5 |
| Alumine m ultrapure $\sigma$ | 39 2,5 | 48 2,5 | 13 2,5 |
| Alumine m + 3 000 ppm Fe$_3$O$_4$ $\sigma$ | 29 1,5 | 40 2,5 | 31 2,5 |

La présence d'un métal de transition dans la matrice affecte sensiblement les résultats.

**Exemples 9 à 13**

Le même résidu Koweit a été analysé selon les conditions de l'exemple 1 mais en l'absence de matrice, la goutte d'échantillon étant placée directement dans la nacelle.

Les résultats sont présentés dans le tableau III et ne sont manifestement pas répétables.

**Tableau III**

| | 600⁻ % | Pyrolysable % | Résidu % |
|---|---|---|---|
| | 38,2 | 60,0 | 1,8 |
| Absence de | 60,6 | 29,4 | 10,0 |
| support | 56,4 | 32,5 | 11,1 |
| | 53,1 | 41,7 | 5,2 |
| | 54,0 | 25,5 | 20,5 |

**Exemples 14, 15, 16, 17**

On reprend l'exemple 6 où l'on opère en présence de silice pure dans un rapport pondéral de 0,2 mg/mg de silice que l'on compare aux exemples ci-dessous où l'on modifie le rapport poids d'hydrocarbures - poids de silice. Les résultats illustrés dans le tableau IV, ne sont ni répétables ni comparables avec ceux de l'exemple 6.

**Tableau IV**

| Exemples | Hydrocarbures $SiO_2$ | 600⁻ % | Pyrolysable % | Résidu % |
|---|---|---|---|---|
| 14 | 0,008 | 52 | 43 | 5 |
| 15 | 0,008 | 40 | 45 | 15 |
| 16 | 1,2 | 48 | 45 | 7 |
| 17 | 1,2 | 37 | 40 | 23 |
| 6 | 0,2 | 43 | 47 | 10 |

**Exemples 18, 19, 20**

Les trois résidus selon les exemples 1, 2 et 3 et analysés selon l'invention sont comparés dans le tableau V aux valeurs de carbone Conradson (CCR) obtenues selon la norme ASTM D 189. On rappelle que la détermination du carbone résiduel obtenu selon l'invention est égale au produit de la teneur en résidu tel que décrit selon l'invention par le facteur 0,83 et peut alors être comparée à la valeur du carbone Conradson.

**Tableau V**

| | Résidu % poids | carbone résiduel % poids | CCR % poids |
|---|---|---|---|
| Safaniya | 12,0 | 10 ± 1,0 | 12,0 ± 1,2 |
| Safaniya hydrotraité | 7,5 | 6,4 ± 0,5 | 5,6 ± 0,6 |
| Boscan | 13,2 | 11,0 ± 1,0 | 9,7 ± 1,0 |

**Revendications**

1. Procédé de détermination de la composition d'un mélange d'hydrocarbures en fonction de la température d'ébullition de ses constituants comprenant une étape d'évaporation et une étape de pyrolyse en atmosphère inerte, *caractérisé* en ce que:

a) on imprègne une matrice poreuse, stable et inerte par lesdits hydrocarbures dans un rapport pondéral de 0,01 à 1 mg d'hydrocarbures par milligramme de matrice,

b) on chauffe à température progressivement croissante et sous flux d'atmosphère inerte la matrice ainsi imprégnée jusqu'à une température $\theta_3$ comprise entre 500 et 600°C de façon à réaliser durant le temps de montée à ladite température ladite étape d'évaporation et ladite étape de pyrolyse sur au moins une partie desdits hydrocarbures et l'on produit un effluent d'évaporation, un effluent de pyrolyse et un résidu,

c) on mesure sur lesdits effluents d'évaporation et de pyrolyse au cours de ce chauffage et au moyen d'un détecteur d'hydrocarbures, au moins deux signaux $Q_1....Q_n$ représentatifs d'une quantité d'hydrocarbures, lesdits signaux correspondant à au moins deux intervalles de temps définis successifs de ce chauffage,

d) on convertit lesdits intervalles de temps en intervalles de températures d'ébullition, et

e) on détermine à partir des signaux $Q_1...Q_n$ et des intervalles de températures d'ébullition la composition du mélange d'hydrocarbures.

2. Procédé selon la revendication 1, dans lequel on réalise, en outre sur ledit résidu, une combustion à une température $\theta_4$ supérieure à la température $\theta_3$ sous courant de gaz oxydant, ladite combustion produisant un effluent gazeux de combustion et on mesure sur ledit effluent gazeux de combustion au moyen d'au moins un détecteur approprié un signal R représentatif de la quantité dudit résidu et on détermine à partir des mesures des signaux $Q_1...Q_n$ et des intervalles de températures d'ébullition et à partir de la mesure du signal R la composition du mélange d'hydrocarbures.

3. Procédé selon les revendications 1 ou 2, dans lequel, dans l'étape b), on chauffe ladite matrice imprégnée jusqu'à une température finale $\theta_2$ correspondant à ladite étape d'évaporation comprise entre 330 et 370°C puis jusqu'à une température finale $\theta_3$ correspondant à ladite étape de pyrolyse comprise entre 500 et 600°C et dans lequel, dans l'étape c), on mesure au moins un signal $Q_1$ représentatif d'une quantité d'hydrocarbures correspondant à au moins un intervalle de temps défini de ladite étape d'évaporation et au moins un signal $Q_n$ représentatif d'une quantité d'hydrocarbures correspondant à au moins un intervalle de temps défini successif de ladite étape de pyrolyse.

4. Procédé selon l'une des revendications 1 à 3 dans lequel la matrice est un oxyde non réductible contenant moins de 1 000 ppm (parties par million) de métaux de transition.

5. Procédé selon l'une des revendications 1 à 4 dans lequel on chauffe la matrice imprégnée à une vitesse d'augmentation de température de 5 à 50°C par minute durant les étapes d'évaporation et de pyrolyse.

6. Procédé selon l'une des revendications 1 à 5 dans lequel on imprègne la matrice dans un rapport pondéral de 0,04 à 0,8 mg d'hydrocarbures par milligramme de matrice.

7. Procédé selon l'une des revendications 1 à 6 dans lequel on détermine des intervalles de temps compris entre 1 et 120 secondes.

8. Procédé selon l'une des revendications 1 à 7 dans lequel on applique des paliers isothermes d'une durée comprise entre 0,5 et 20 minutes au début et à la fin des étapes d'évaporation et de pyrolyse.

9. Procédé selon l'une des revendications 1 à 8 dans lequel on utilise lors du chauffage un flux de gaz inerte de débit spécifique rapporté à la quantité de matrice poreuse imprégnée, de 1 à 50 millilitres par milligramme et par minute.

10. Procédé selon l'une des revendications 1 à 9 dans lequel on réalise ladite combustion durant 2 à 20 minutes.

11. Procédé selon l'une des revendications 1 à 10 dans lequel la matrice est une alumine, une silice ou une silice-alumine.


**Patentansprüche**

1. Verfahren zum Bestimmen der Zusammensetzung eines Gemisches aus Kohlenwasserstoffen als Funktion der Siedetemperatur seiner Bestandteile, mit einer Verdampfungsstufe und einer Pyrolysestufe in inerter Atmosphäre, *dadurch gekennzeichnet,* daß

a) man eine poröse, stabile und inerte Matrix durch diese Kohlenwasserstoffe in einem Gewichtsverhältnis von 0,01 bis 1 mg Kohlenwasserstoffe pro Milligramm Matrix imprägniert,

b) man bei allmählich steigender Temperatur und unter einem Strom aus inerter Atmosphäre die so imprägnierte Matrix bis auf eine Temperatur $\theta_3$ erwärmt, die zwischen 500 und 600°C liegt, derart, daß während der Zeit des Anstiegs auf diese Temperatur diese Verdampfungsstufe und diese Pyrolysestufe an wenigstens einem Teil dieser Kohlenwasserstoffe realisiert wird und man einen Verdampfungsabstrom, einen Pyrolyseabstrom und einen Rückstand erzeugt,

c) man an diesen Verdampfungs- und Pyrolyseabströmen während dieser Erwärmung und mittels eines Kohlenwasserstoffdetektors wenigstens zwei Signale $Q_1....Q_n$ mißt, die repräsentativ für eine Kohlenwasserstoffmenge sind, wobei diese Signale wenigstens zwei definierten aufeinanderfolgenden Zeitintervallen dieser Erwärmung entsprechen,

d) man wandelt diese Zeitintervalle in Siedetemperaturintervalle um, und

e) man bestimmt ausgehend von den Signalen $Q_1....Q_n$ und den Siedetemperaturintervallen die Zusammensetzung des Kohlenwasserstoffgemisches.

2. Verfahren nach Anspruch 1, bei dem man ausser diesem Rückstand eine Verbrennung bei einer Temperatur $\theta_4$, die größer als die Temperatur $\theta_3$ ist, unter einem Strom oxidierenden Gases realisiert, wobei diese Verbrennung einen gasförmigen Verbrennungsabstrom erzeugt und bei dem man an diesem Verbrennungsabstrom vermittels wenigstens eines geeigneten Detektors ein Signal R mißt, das repräsentativ für die Menge dieses Rückstandes ist und man ausgehend von den Messungen der Signale $Q_1....Q_n$ und den Siedetemperaturintervallen und ausgehend von der Messung des Signals R die Zusammensetzung des Kohlenwasserstoffgemisches bestimmt.

3. Verfahren nach den Ansprüchen 1 und 2, bei dem in Stufe b) man diese imprägnierte Matrix bis auf eine Endtemperatur $\theta_2$ entsprechend dieser Verdampfungsstufe zwischen 330 und 370°C, dann bis auf eine Endtemperatur $\theta_3$ entsprechend dieser Pyrolysestufe zwischen 500 und 600°C erwärmt und bei dem in Stufe c) man wenigstens ein Signal $Q_1$ mißt, welches repräsentativ für eine Menge an Kohlenwasserstoffen ist, die wenigstens einem definierten Zeitintervall dieser Verdampfungsstufe entspricht und wenigstens ein Signal $Q_n$ mißt, welches repräsentativ für eine Kohlenwasserstoffmenge entsprechend wenigstens einem definiert nachfolgenden Zeitintervall dieser Pyrolysestufe entspricht.

4. Verfahren nach einem der Ansprüche 1 bis 3, bei dem die Matrix ein nicht reduzierbares Oxid ist, das wenigstens 1 000 ppm (Teile pro Million) an Übergangsmetallen enthält.

5. Verfahren nach einem der Ansprüche 1 bis 4, bei dem man die imprägnierte Matrix bei einer Temperaturerhöhungsgeschwindigkeit von 5 bis 50°C pro Minute während der Verdampfungs- und Pyrolysestufen erwärmt.

6. Verfahren nach einem der Ansprüche 1 bis 5, bei dem man die Matrix in einem Gewichtsverhältnis von 0,04 bis 0,8 mg Kohlenwasserstoffe pro Milligramm Matrix imprägniert.

7. Verfahren nach einem der Ansprüche 1 bis 6, bei dem man Zeitintervalle zwischen 1 und 120 Sekunden bestimmt.

8. Verfahren nach einem der Ansprüche 1 bis 7, bei dem man isotherme Stufen von einer Dauer zwischen 0,5 und 20 Minuten bei Beginn und Ende der Verdampfungs- und Pyrolysestufen anwendet.

9. Verfahren nach einem der Ansprüche 1 bis 8, bei dem man bei der Erwärmung einen Inertgasstrom spezifischer Menge, bezogen auf die Menge an poröser imprägnierter Matrix, von 1 bis 50 Milliliter pro Milligramm und Minute verwendet.

10. Verfahren nach einem der Ansprüche 1 bis 9, bei dem man die Verbrennung 2 bis 20 Minuten lang durchführt.

11. Verfahren nach einem der Ansprüche 1 bis 10, bei dem die Matrix ein Aluminiumoxid, ein Siliciumoxid und ein Siliciumoxid-Aluminiumoxid ist.


## Claims

1. A process for determining the composition of a hydrocarbon mixture from the boiling temperature of its constituents, comprising an evaporation step and a pyrolysis step in inert atmosphere, characterized by the following operations of:

a) impregnating a stable, inert and porous matrix with said hydrocarbons in a proportion by weight from 0.01 to 1 mg of hydrocarbons per milligram of matrix,

b) heating the so-impregnated matrix at a progressively increasing temperature under a flow of inert atmosphere up to a temperature $\theta_3$ ranging from 500 to 600°C, so as to achieve, during said temperature increase, an evaporation step and a pyrolysis step on at least a portion of said hydrocarbons, thus producing an evaporation effluent, a pyrolysis effluent and a residue,

c) measuring, during said heating step on said effluents, by means of a hydrocarbon detector, at least two signals $Q_1...Q_n$ representative of a hydrocarbon amount, said signals corresponding to at least two successive defined time intervals of said heating step,

d) converting said time intervals to boiling temperature interval, and

e) determining the composition of hydrocarbon mixture from signals $Q_1...Q_n$ and boiling temperature intervals.

2. A process according to claim 1 wherein subjecting moreover said residue to a combustion at a temperature $\theta_4$ higher than temperature $\theta_3$, under a stream of oxidizing gas, said combustion producing a combustion gas effluent, measuring on said combustion gaseous effluent by means of at least one suitable detector, a signal R representative of the residue amount and determining from the measured values of signals $Q_1...Q_n$ and the boiling temperature intervals and from the measure of signal R the composition of hydrocarbon mixture composition.

3. A process according to claims 1 or 2, where, in step (b) the impregnated matrix is heated up to a final temperature $\theta_2$ corresponding to said evaporation step, ranging from 330 to 370°C, and then up to a final temperature $\theta_3$ corresponding to said pyrolysis step, ranging from 500 to 600°C, and wherein step (c) comprises the measurement of at least one signal $Q_1$ representative of a hydrocarbon amount corresponding to at least one defined time interval of said evaporation step and of at least one signal $Q_n$ representative of a hydrocarbon amount corresponding to at least one defined time interval following said pyrolysis step.

4. A process according to one of claims 1 to 3 , wherein the matrix is a non reducible oxide containing less than 1 000 ppm (parts per million) of transition metals.

5. A process according to one of claims 1 to 4, wherein the impregnated matrix is heated at a rate of temperature increase from 5 to 50°C per minute during the evaporation and pyrolysis steps.

6. A process according to one of claims 1 to 5, wherein the matrix is impregnated with hydrocarbons in a proportion by weight from 0.04 to 0.8 mg of hydrocarbons per milligram of matrix.

7. A process according to one of claims 1 to 6, wherein time intervals range from 1 to 120 seconds.

8. A process according to one of claims 1 to 7, wherein isothermal stages lasting from 0.5 to 20 minutes at the beginning and at the end of the evaporation and pyrolysis steps are applied.

9. A process according to one of claims 1 to 8, wherein, during the heating step, a flow of inert gas is used at a specific flow rate of 1 - 50 millimeters per milligram of impregnated porous matrix and per minute.

10. A process according to one of claims 1 to 9, wherein said combustion lasts 2 - 20 minutes.

11. A process according to one of claims 1 to 10, wherein the matrix is an alumina, a silica or a silica-alumina.

**FIG.1**

FIG.2A

FIG.2B

FIG.3